# EUROPÄISCHE PATENTANMELDUNG

(11) **EP 0 774 246 A2**
(43) Veröffentlichungstag der Anmeldung: **21.05.1997**
(21) Anmeldenummer: 96118319.1
(22) Anmeldetag: 15.11.1996
(51) Int. Cl.: A61H 23/02

(54) **Massagevorrichtung**

(30) Priorität: 18.11.1995 DE 19543108
(71) Anmelder: Heddernheimer Metallwarenfabrik GmbH, D-78464 Konstanz (DE)
(72) Erfinder:
(74) Vertreter: Patentanwälte Ruff, Beier, Schöndorf und Mütschele

(57) **Zusammenfassung**

Eine Massagevorrichtung besitzt eine mattenartige Auflage (2), Mittel zur Erzeugung von Vibrationsbewegungen in der Auflage und zusätzliche separate Auflageelemente (3, 4, 5, 6) für mindestens einen Teil der zu massierenden Körperbereiche. Diese Auflageelemente sind an der Auflage einzeln anordenbar, vorzugsweise in Längs- und/oder Querrichtung verschiebbar. Unterschiedliche Auflageelemente sind entsprechend der zu massierenden Körperbereiche ausgebildet.

Zusätzlich können integrierte Mittel zur Magnetbehandlung, zur Infrarotbehandlung und/oder Heizmittel vorgesehen sein.

## Beschreibung

Die Erfindung betrifft eine Vorrichtung und ein Verfahren zur Massagebehandlung.

Aufgrund des stetig wachsenden Gesundheitsbewußtseins und dem Wunsch nach Entspannung gibt es bereits eine Vielzahl von Massagevorrichtungen, die sowohl von einem Therapeuten als auch vom Benutzer zuhause angewandt werden können. Darunter befinden sich auch sog. Massageliegen oder Massagematten. Derartige Vorrichtungen haben jedoch oft den Nachteil, daß sie an die Körpergröße oder Körperform der Benutzer nicht anpaßbar sind. Dadurch erfolgt die Massage zumindest teilweise nicht gezielt an den Körperbereichen, die massiert werden sollen. Außerdem sind derartige Matten oder Liegen vergleichsweise sperrig, d.h. sie nehmen insbesondere bei Transport und Aufbewahrung einen großen Stauraum in Anspruch.

Die Erfindung stellt sich die Aufgabe, die genannten und weitere Nachteile bekannter Massageliegen oder Massagematten zu vermeiden. Insbesondere soll eine Massagevorrichtung zur Verfügung gestellt werden, die in einfacher Weise an die Körpergröße und Körperform verschiedener Benutzer anpaßbar ist und gleichzeitig einen platzsparenden Transport und Aufbewahrung der Vorrichtung möglich macht.

Diese Aufgabe wird gelöst durch die Massagevorrichtung mit den Merkmalen des Anspruchs 1. Bevorzugte Ausführungsformen sind in den Unteransprüchen beschrieben. Ein entsprechendes Verfahren zur Massagebehandlung zeigt Anspruch 13. Der Wortlaut sämtlicher Ansprüche wird durch Bezugnahme zum Inhalt dieser Beschreibung gemacht.

Erfindungsgemäß besitzt die Massagevorrichtung
- eine mattenartige Auflage,
- Mittel zur Erzeugung oder Aufbringung von Vibrationsbewegungen in bzw. auf die mattenartige Auflage, und
- zusätzliche separate Auflageelemente für mindestens einen Teil der zu massierenden Körperbereiche, wobei diese Auflageelemente an der Auflage anordenbar sind.

Die mattenartige Auflage ist für den Benutzer bzw. für einen Teil seines Körpers vorgesehen. Üblicherweise wird sich der Benutzer auf die Auflage legen. Bei den zusätzlichen Auflageelementen, von denen erfindungsgemäß mindestens eines vorhanden ist, handelt es sich um von der mattenartigen Auflage getrennte Elemente, die je nach Bedarf einzeln oder zu mehreren an der Oberseite und/oder der Unterseite der mattenartigen Auflage anordenbar sind. Die Auflageelemente sind üblicherweise nach Art eines Kissens ausgebildet.

Durch die beschriebene Merkmalskombination, insbesondere durch die Trennung von mattenartiger Auflage und Auflageelementen ist eine einfache Anpassung der Massagevorrichtung an Körpergröße/Körperform des Benutzers möglich. Gleichzeitig läßt sich die Vorrichtung platzsparend transportieren und aufbewahren, da durch die separaten Auflageelemente eine niedrigere Ausführung der mattenartigen Auflage ermöglicht wird. Außerdem hat die erfindungsgemäße Vorrichtung den Vorteil, daß durch die Auflageelemente eine flächige Übertragung der erzeugten Vibrationsbewegungen auf die zu massierenden Körperbereiche erfolgt oder durch insbesondere anatomisch geformte, separate Auflageelemente eine gezielte und sogar punktuelle Massage erreicht werden kann (Fakireffekt).

Bei der Erfindung kann die mattenartige Auflage insbesondere zusammengefaltet werden. Vorzugsweise ist sie zusammenrollbar. Dadurch werden Transport und/oder Lagerung weiter vereinfacht. Außerdem kann die mattenartige Auflage aufgrund der vorzugsweise vorhandenen Flexibilität in einfacher Weise in ein Bett, einen Stuhl o.dgl. eingelegt werden, so daß auch in diesen Möbeln und nicht nur auf dem Boden oder einer harten Unterlage die Massagebehandlung durchgeführt werden kann.

Bei bevorzugten Ausführungsformen sind die (separaten) Auflageelemente an der (mattenartigen) Auflage in Längs- und/oder Querrichtung verschiebbar anordenbar. Die entsprechende Verschiebung kann dabei entweder völlig frei oder auch mit Hilfe geeigneter Führungsmittel, wie beispielsweise Schnüren, Bändern oder dergleichen, erfolgen. Die Verschiebbarkeit erleichtert die Anpassung an Körpergröße/Körperform des Benutzers.

In Weiterbildung sind die Auflageelemente an der Auflage, d.h. an deren Ober- oder Unterseite, lösbar festlegbar. Durch diese Festlegung wird verhindert, daß sich die Auflageelemente in unerwünschter Weise verschieben. Die angestrebte Festlegung kann beispielsweise durch eine geeignete Auswahl des Oberflächenmaterials der Auflageelemente erreicht werden, welches durch Reibung eine weitere Verschiebung des Auflageelementes entlang oder quer zur Auflage verhindert. Alternativ oder zusätzlich können Befestigungsmittel nach Art von Bändern oder Riemen vorgesehen sein, die beispielsweise in Querrichtung um die Auflage gelegt und an dieser oder untereinander zum Beispiel durch Druckknöpfe oder textile Haftverschlüsse ("Klettverschluß") befestigbar sind. Selbstverständlich können die Auflageelemente auch direkt mit Hilfe von textilen Haftverschlüssen oder Druckknöpfen an der mattenartigen Auflage festgelegt werden.

Die separaten Auflageelemente (Auflagekissen, Auflagepolster) sind vorzugsweise körpergerecht gestaltet, d.h. sie sind entsprechend dem zu massierenden Körperbereich ("anatomisch") ausgebildet. Dies ermöglicht eine weitgehende Optimierung und Abstimmung der Massage auf die einzelnen Körperbereiche.

Grundsätzlich ist es möglich, ein oder mehrere Auflageelemente vorzusehen, die gleichzeitig für die Behandlung verschiedener zu massierender Körperbereiche geeignet sind, beispielsweise dadurch, daß sie mit unterschiedlicher Orientierung an der Auflage anordenbar sind. Es ist jedoch bevorzugt, wenn zumindest für die wichtigsten zu massierenden Körperbereiche jeweils mindestens ein separates Auflageelement vorgesehen ist. Dadurch können die Vibrationsbewegungen gezielt auf die jeweiligen Körperbereiche abgestimmt werden. Gegebenenfalls kann auch das gleiche Auflageelement in zwei oder mehr verschiedenen Orientierungen zur Massage desselben Körperbereichs vorgesehen sein. Dementsprechend sind erfindungsgemäß insbesondere jeweils ein Auflageelement für die folgenden Körperbereiche/Körperzonen vorhanden:
- den Schulterbereich, ggf. einschließlich Kopf- und Nackenbereich,
- den Bereich unterer Rücken/Gesäß/Oberschenkel,
- den Unterschenkelbereich,
- ggf. den Fußbereich oder Fußsohlenbereich.

Die vorstehenden Angaben definieren die Bereiche, für die die einzelnen Auflageelemente im wesentlichen vorgesehen sind. So kann beispielsweise ein Auflageelement im wesentlichen nur für den Bereich der Schultern und Schulterblätter, d. h. für den Bereich des "oberen Rückens" vorgesehen sein. Ein derartiges Auflageelement kann jedoch bei einer anderen Ausführung gleichzeitig auch zur Auflage und damit gegebenenfalls auch zur Massage des Kopf- und Nackenbereichs vorgesehen sein. Deshalb ist der Begriff "Schulterbereich" sowohl in den Ansprüchen als auch in der Beschreibung nicht beschränkend zu verstehen, sondern soll entsprechend erweiterte Ausführungen wie sie soeben beschrieben wurden, umfassen. Entsprechendes gilt für die anderen Definitionen der Körperbereiche wie sie bei der Erfindung angegeben sind.

Die Form des Auflageelementes, d.h. beispielsweise Grundfläche, Dicke, Material des entsprechenden Kissens, ist vorzugsweise an den zu behandelnde Körperbereich angepaßt. Dies führt dazu, daß bei bevorzugten Ausführungsformen die Auflageelemente für den Schulterbereich (gegebenenfalls Kopf-/Nacken-/Schulterbereich) und/oder den Unterschenkelbereich eine trapezartige Form (Grundfläche des Kissens) besitzen. Ein entsprechendes Auflageelement für den Bereich unterer Rücken/Gesäß/Oberschenkel besitzt eine Form nach Art eines Doppeltrapezes. Ein Auflageelement für den Fußbereich, insbesondere zur Massage der Fußsohlen, ist nach Art eines Keils/Wulstes aufgebaut, wobei die zur Auflage der Fußsohlen bestimmte Fläche zur Unterstützung der Massagefunktion eine rippenartige Struktur besitzen kann.

Sowohl die mattenartige Auflage als auch die separaten Auflageelemente bestehen üblicherweise aus einem Bezug und einer darin enthaltenen Füllung. Die Materialien für Bezug und Füllung können je nach Anwendung variiert werden. So finden als Füllmaterial vorzugsweise Schaumstoffe, insbesondere Schaumkunststoffe wie Polyurethane u.a., Verwendung. Derartige Schaumstoffe oder auch Gummi sind besonders geeignet, die Vibrationsbewegungen auf den Körper der zu behandelnden Person zu übertragen. Gegebenenfalls können andere als die erwähnten Materialien verwendet werden. Beispielsweise ist es möglich, als Füllmaterial zumindest teilweise Latex zu verwenden. Weiter ist es denkbar, zusätzlich Luft in die Auflageelemente einzubringen, oder diese nach dem Luftkammerprinzip auszugestalten, um eine bequeme Auflage zu erreichen. Das Einbringen von Luft kann ggf. auch durch den Benutzer selbst erfolgen.

Je nach Anwendung und/oder erwünschter Massagewirkung können die mattenartige Auflage und die Auflageelemente aus unterschiedlich geformtem und/oder unterschiedlich hartem Material, insbesondere entsprechendem Schaumstoffmaterial gebildet sein. Gegebenenfalls ist es auch möglich, die einzelnen Auflageelemente aus solchen unterschiedlich geformten und/oder unterschiedlich harten Materialien auszubilden. Durch die genannten Maßnahmen wird beispielsweise erreicht, daß der Liegekomfort für den Benutzer oder die Massagewirkung entweder durch den Benutzer oder bereits werksseitig gezielt an die einzelnen Körperbereiche angepaßt werden kann.

Das Material des Bezuges besteht üblicherweise aus Textilien, Leder oder Kunstleder, um einen für den Benutzer angenehmen Kontakt herzustellen. Vorzugsweise werden dabei abwischbare oder abwaschbare Materialien verwendet, um eine einfache Reinigung zu ermöglichen.

Grundsätzlich können die Vibrationsbewegungen auf oder in die Auflage durch alle geeigneten entsprechenden Mittel erzeugt werden, die sich auch außerhalb der Auflage selbst befinden können. Erfindungsgemäß ist es bevorzugt, wenn sich diese Mittel an oder in der Auflage selbst befinden, wobei sie vorzugsweise in die Auflage integriert, d.h. beispielsweise in das Schaumstoff-Füllmaterial eingebracht sind. Vorzugsweise handelt es sich bei den Mitteln zur Erzeugung von Vibrationsbewegungen um mindestens einen Elektromotor, der durch seine Konstruktion oder durch zusätzliche Einrichtungen Vibrationen auf die Auflage und dann entweder direkt oder durch die als Übertragungselemente dienenden Auflageelemente auf den Benutzer überträgt. Insbesondere kann der Elektromotor ein Exzentergewicht besitzen, das auf seiner Antriebswelle befestigt ist.

Bei weiteren bevorzugten Ausführungsformen weist nicht nur die mattenartige Auflage, sondern auch mindestens eines der separaten Auflageelemente Mittel zur Erzeugung oder zum Aufbringen von Vibrationsbewegungen, d.h. beispielsweise einen der beschriebenen Elektromotoren auf. Damit wird bei einer üblichen Anordnung der Auflageelemente an der Ober- oder Unterseite der Auflage die Massagewirkung verstärkt. Außerdem wird durch derartige Ausbildungen eine weitere Massageart ermöglicht, nämlich diejenige, bei der der Benutzer mindestens eines der beschriebenen Auflageelemente an der Körperseite auf- oder anlegt, die der mattenartigen Auflage abgewandt ist. Damit wird eine "beidseitige" Massage ermöglicht, die auch als "Sandwich-Massage" bezeichnet werden kann. So kann beispielsweise ein Auflageelement für den Bereich unterer Rücken/Gesäß/Oberschenkel, das Mittel zur Erzeugung von Vibrationsbewegungen aufweist, auf den Bauchbereich aufgelegt werden, so daß bei Rückenlage des Benutzers auf der Auflage eine gleichzeitige Massage von Schulterbereich, Bauchbereich, Unterschenkelbereich und ggf. Fußbereich möglich wird. Die genannten Auflageelemente können ggf. auch in Verbindung mit anderen Unterlagen, die keine Vibrationsbewegungen erzeugen können oder als reine Massagekissen Verwendung finden.

Wie beschrieben kann ein Auflageelement für den Bereich von Kopf/Nacken/Schulter (oberer Rücken) des Benutzers vorgesehen sein. Zur Unterstützung des Kopf- und/oder Nackenbereiches vor, während oder nach der Massage kann die Vorrichtung alternativ oder zusätzlich ein geeignetes Kopf- oder Nackenkissen (-polster, -rolle) aufweisen. Dieses kann ggf. auch Mittel zur Erzeugung von Vibrationsbewegungen enthalten, was üblicherweise nicht vorgesehen ist. Ein derartiges Kopf- oder Nackenkissen kann mit Hilfe geeigneter Befestigungsmittel wie beispielsweise Riemen oder Bändern an der Massagevorrichtung unverlierbar befestigt sein, wie dies beispielsweise von Auflagen für Gartenstühle oder -liegen bekannt ist. Die Bänder können beispielsweise durch Druckknöpfe oder textile Haftverschlüsse an der mattenartigen Auflage festgelegt sein.

Zur Variation der Behandlung oder zur Unterstützung der Massagewirkung durch weitere therapeutisch erwünschte Effekte können bei weiteren Ausführungsformen der Erfindung in/an der Auflage oder in/an mindestens einem Auflageelement Mittel zur Magnetbehandlung, zur Infrarottherapie oder Heizmittel vorgesehen sein.

Mittel zur Magnetbehandlung können positive Auswirkungen auf den Benutzer besitzen, da sie beispielsweise den Ionenhaushalt positiv beeinflussen können. Zu diesem Zweck können Permanentmagneten, wie beispielsweise Magnetfolien oder pulsgesteuerte Magneten mit wechselnden Magnetfeldern, vorhanden sein.
Bei den Mitteln zur Infrarotbehandlung können Niedertemperatur-Lampen vorhanden sein, die mit Hilfe sog. Tiefenwärme positive Auswirkungen auf den Benutzer haben.

Bei den erwähnten Heizmitteln kann es sich um übliche Widerstandsheizungen handeln, die eine Erwärmung der Auflage oder des Auflageelementes bewirken und somit einen wohltuenden Effekt auf den Benutzer ausüben. Auf diese Weise kann die Vorrichtung beispielsweise vor der Massage auf eine angenehme Temperatur gebracht werden.

Schließlich weist die erfindungsgemäße Vorrichtung bei bevorzugten Ausführungen ein Bedienelement zur Steuerung der verschiedenen möglichen Funktionen auf. Dabei handelt es sich vorzugsweise um eine Fernbedienung, die ggf. auch drahtlos ausgebildet sein kann. Insbesondere sind durch das Bedienelement die Parameter einer Massagebehandlung anhand vordefinierter und/oder individuell einstellbarer Werte auswählbar. Parameter einer solchen Behandlung sind beispielsweise Zeit und Stärke (Intensität) der Behandlung. Weiter können über die vorhandenen Vibrationserzeugungsmittel Rhythmus, Intervall, Periode, Richtung, Bereich (Zone), Fläche usw. der Massagebehandlung variiert werden. Dementsprechend können mit Hilfe des Bedienelementes Massagen nach einer bestimmten vorgegebenen Grundeinstellung oder nach bestimmten vorgegebenen Programmen durchgeführt werden, bei denen üblicherweise die vorhandenen Vibrationserzeugungsmittel gleichzeitig oder einzeln in beliebiger Reihenfolge oder Dauer ansteuerbar sind. Zu diesem Zweck ist im Bedienelement üblicherweise ein sog. "Mikrocomputer" (Prozessor) vorhanden.

Bei bevorzugten Ausführungsformen kann die Auflage durch geeignete Anordnung der Vibrationserzeuger in vier Massagezonen eingeteilt sein. Jede Anzahl von Massagezonen kann mit Hilfe des Bedienelements gezielt in bestimmter Reihenfolge oder Kombination angesteuert werden. Dabei ist es möglich, dem Benutzer bestimmte voreingestellte Programmabläufe zur Verfügung zu stellen oder ihm eine völlig selbständige Variation zu ermöglichen. Darüber hinaus können die Vibrationserzeuger, insbesondere die entsprechenden Elektromotoren, in verschiedener Weise zur Erzeugung von bestimmten Massagearten angesteuert werden, beispielsweise in kontinuierlichem Betrieb zur Erzeugung einer Dauervibration oder in stoßartigem Betrieb zur Erzeugung einer oszillierenden Vibration. Die einzelnen Betriebsarten können sowohl hinsichtlich der Ansteuerung der Vibrationserzeuger (Vibration, Oszillation) als auch bezüglich der Zuschaltung der Massagezonen und beispielsweise der Stärke (Intensität) beliebig überlagert werden, so daß sich hier weitere Variationsmöglichkeiten ergeben.

Das erfindungsgemäße Verfahren ist dadurch gekennzeichnet, daß die in einer mattenartigen Auflage mit Hilfe von geeigneten Mitteln erzeugten Vibrationsbewegungen durch zusätzliche separate Auflageelemente, die an der mattenartigen Auflage anordenbar sind, auf den Körper des Benutzers übertragen werden. Dadurch wird eine flächige Übertragung bzw. gezielte punktuelle Massage zumindest an den Teilen der zu massierenden Körperbereiche erreicht, für die entsprechende Auflageelemente vorgesehen sind. Weitere Ausgestaltungmöglichkeiten des erfindungsgemäßen Verfahrens ergeben sich aus der bisherigen Beschreibung und der folgenden Figurenbeschreibung.

Die beschriebenen Merkmale und weitere Merkmale der Erfindung ergeben sich aus der nachfolgenden Beschreibung einer bevorzugten Ausführungsform in Verbindung mit den Unteransprüchen und den Zeichnungen. Hierbei können die einzelnen Merkmale jeweils für sich oder zu mehreren in Kombination miteinander verwirklicht sein.

In den Zeichnungen zeigen:
- Fig. 1: eine schematische Darstellung einer erfindungsgemäßen Vorrichtung in Draufsicht, und
- Fig. 2: eine schematische Darstellung einer erfindungsgemäßen Vorrichtung in Seitenansicht.

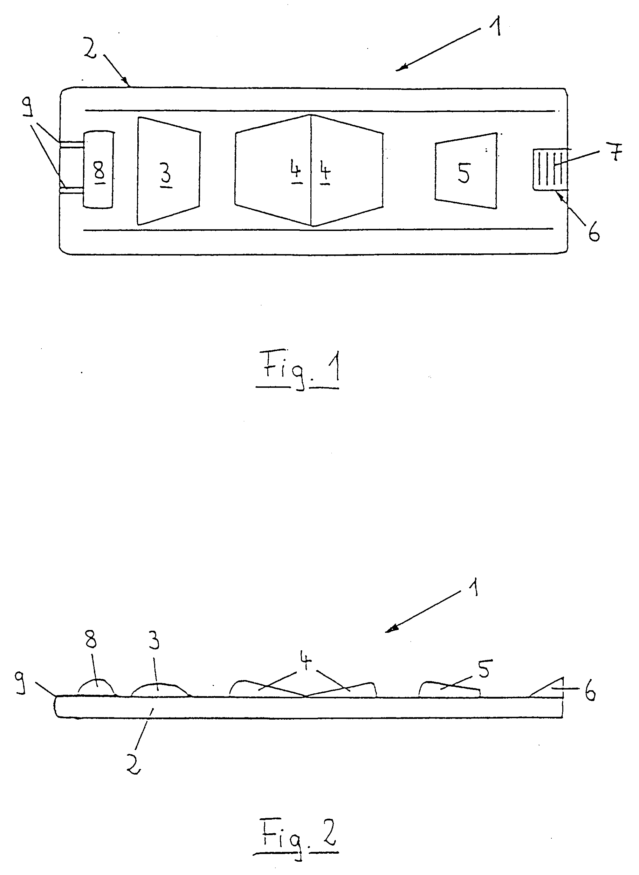

Gemäß Fig. 1 besitzt die Massagevorrichtung 1 eine Auflage 2 in Form einer Matte. In das Innere der Matte integriert sind Mittel zur Erzeugung von Vibrationsbewegungen, nämlich entsprechend konstruierte Elektromotoren, die in Fig. 1 nicht dargestellt sind.

Auf der Oberseite der Auflage 2, auf die sich ein Benutzer bei Betrieb der Vorrichtung 1 legt, sind verschiedene separate Auflageelemente 3 bis 6 angeordnet. Die dargestellten Ecken können abgerundet sein. Im einzelnen handelt es sich um
- ein trapezförmiges Auflageelement 3 nach Art eines Kissens für den Schulterbereich des Benutzers,
- ein Auflageelement 4 in Form eines Doppeltrapzes, ebenfalls nach Art eines Kissens für die Körperbereiche unterer Rücken, Gesäß und Oberschenkel,
- ein trapezförmiges Auflageelement 5 nach Art eines Kissens für den Unterschenkel (Waden-)Bereich, und
- ein keilförmiges Auflageelement 6 für die Füße, insbesondere Fußsohlen, des Benutzers, bei dem zusätzlich Vertiefungen bzw. Rippen 7 zur Ausübung und Unterstützung einer Fußsohlenreflexzonen-Massage vorgesehen sind. Wie beschrieben kann ein solches Element auch wulstförmig, d. h. beispielsweise mit halbrundem Querschnitt ausgebildet sein.

Das Auflageelement 4 kann, wie in Fig. 1 angedeutet, entlang seiner Mitte in entsprechender Weise abgesteppt und damit zusammenfaltbar sein. Dadurch kann es auch in zusammengelegtem Zustand als Unterlage, beispielsweise für den Kopf-/Schulterbereich oder Unterschenkel-/Fußbereich verwendet werden. In Abweichung von der dargestellten Form kann die Doppeltrapezform des Auflagelementes 4 auch so verwirklicht sein, daß dieses an seinen beiden Stirnenden breiter ist als in der Mitte, d. h. eine "knochenartige" Form besitzt. Ein derartiges Auflageelement kann gegebenenfalls auch quer auf die Auflage 2 aufgelegt sein, d. h. gegenüber der Darstellung von Fig. 1 um 90° gedreht.

An dem Ende der Auflage 2, das dem Kopf des Benutzers zugeordnet ist, ist ein Kopf- oder Nackenkissen bzw. eine Kopf- oder Nackenstütze 8 vorgesehen, die mit Hilfe von Bändern bzw. Riemen 9 an der Auflage 2 befestigt ist. Die Art dieser Befestigung ist in Fig. 1 nicht näher erläutert und kann beispielsweise mit Hilfe von Druckknöpfen oder textilen Haftverschlüssen erfolgen.

Bei einer in Figur 1 nicht dargestellten Ausführungsform kann das gegebenenfalls trapezförmige Auflageelement 3 zur Auflage von Kopf/Nacken/Schulter (oberer Rücken) vorgesehen sein. Dann kann entweder auf eine Kopf- oder Nackenstütze 8 verzichtet oder diese zusätzlich auf die entsprechende Stelle des Auflageelementes 3 aufgelegt werden.

Wie bereits beschrieben, können die Auflageelemente 3 bis 6 entweder lose auf die Oberseite der Auflage 2 aufgelegt oder lösbar an dieser festgelegt sein, beispielsweise durch eine entsprechende Ausbildung der Unterseite der Auflageelemente oder mit Hilfe von Riemen/Bändern und/oder textilen Haftverschlüssen.

Der jeweilige Benutzer legt dabei die Auflageelemente 3 bis 6 an den Stellen der Auflage 2 auf, die für seine Körpergröße bzw. Körperform passend sind. Vorzugsweise werden die Auflageelemente 3 bis 6 im Wirkungsbereich der nicht dargestellten Vibrationserzeugungsmittel aufgelegt, die jedoch grundsätzlich bereits an den entsprechenden Stellen der Matte bauseitig vorgesehen sind. Gegebenenfalls können an der Matte geeignete Markierungen vorhanden sein, die dem Benutzer entsprechende Anhaltspunkte geben, so daß die Anordnung der Auflageelemente korrekt innerhalb des durch die Elektromotoren vorgegebenen Rasterbereichs erfolgt.

Schließlich ist in Fig. 1 noch dargestellt, daß sich entlang der beiden Längsseiten der Auflage 2 jeweils nicht näher bezeichnete eine Absteppnaht befindet, die für den Benutzer ein bequemes Aufliegen unterstützt. Auch Ziernähte können vorhanden sein. Weiter kann sich an mindestens einer Seitenfläche eine Verschließeinrichtung, wie beispielsweise ein Reißverschluß, befinden, mit dessen Hilfe das Innere der Auflage 2 zugänglich ist. Weiter können sich an den Seitenflächen Griffe, Schlaufen, Schlingen, Bänder oder Riemen befinden, durch die insbesondere zur Aufbewahrung oder zum Transport der Vorrichtung 1 die Auflage 2 und/oder die Auflageelemente 3 bis 6 zusammengebunden oder transportiert werden können. Schlaufen oder Schlingen an den schmalen Seitenflächen können auch zum Aufhängen der Auflage mittels eines Kleiderbügels an einer Garderobe oder in einem Schrank genutzt werden.

Eine übliche Auflagematte 2 kann beispielsweise eine Länge zwischen 180 cm und 200 cm, vorzugsweise 190 cm, und eine Breite zwischen 50 cm und 70 cm, vorzugsweise 60 cm, besitzen. Die Höhe einer solchen Auflage 2 beträgt üblicherweise zwischen 4 und 6 cm, insbesondere ca. 5 cm. Damit kann diese Auflage 2 bei geeigneter Materialwahl einfach zusammengerollt und verstaut werden. Die einzelnen Auflageelemente besitzen entsprechend geringere Abmessungen bzgl. Länge und Breite, deren Verhältnis zur Größe der Auflage 2 näherungsweise aus Fig. 1 hervorgeht. Die gesamte Bauhöhe der erfindungsgemäßen Vorrichtung 1 beträgt bei aufgelegten Auflageelementen üblicherweise zwischen 8 und 12 cm, vorzugsweise ca. 10 cm.

Selbstverständlich sind Abweichungen bei der Form der Auflagematte 2 möglich. So kann beispielsweise eine in Draufsicht tonnenförmige Matte vorgesehen sein, deren im Mittelbereich parallele Seiten zu den Enden hin abgerundet sind.

Fig. 2 zeigt die in Fig. 1 dargestellte Massagevorrichtung 1 in schematischer Seitenansicht, wobei identische Bezugszeichen wie in Fig. 1 verwendet sind. Gemäß Fig. 2 sind die einzelnen Auflageelemente 3 bis 6 und das Kissen 8 in Dicke (Polsterung) und Form an die Form der einzelnen Körperbereiche bzw. -zonen angepaßt, was in Fig. 2 allerdings nur schematisch angedeutet ist. Selbstverständlich ist es möglich, auch andere Formen und Polsterungen der einzelnen Auflageelemente zu verwenden oder entsprechend gestaltete Auflageelemente auch an der Unterseite der Auflage 2 vorzusehen, wie dies bereits beschrieben wurde.

Die Darstellung und Maßverhältnisse der Fig. 1 und 2 sind selbstverständlich für den Gegenstand der Erfindung nicht bindend. Sämtliche Auflageelemente können integrierte Mittel zur Vibrationserzeugung enthalten, wie diese auch in der Auflage 2 selbst vorgesehen sind.

## Patentansprüche

1. Massagevorrichtung mit
- einer mattenartigen Auflage (2),
- Mitteln zum Aufbringen von Vibrationsbewegungen auf die Auflage, und
- zusätzlichen separaten Auflageelementen (3, 4, 5, 6) für mindestens einen Teil der zu massierenden Körperbereiche, die an der Auflage (2) anordenbar sind.

2. Massagevorrichtung nach Anspruch 1, dadurch gekennzeichnet, daß die mattenartige Auflage (2) zusammenlegbar, insbesondere zusammenrollbar, ist.

3. Massagevorrichtung nach Anspruch 1 oder Anspruch 2, dadurch gekennzeichnet, daß die Auflageelemente (3 bis 6) an der Auflage (2) in Längs- und/oder Querrichtung verschiebbar anordenbar sind.

4. Massagevorrichtung nach einem der vorhergehenden Ansprüche, dadurch gekennzeichnet, daß die Auflageelemente (3 bis 6) an der Auflage lösbar festlegbar sind.

5. Massagevorrichtung nach einem der vorhergehenden Ansprüche, dadurch gekennzeichnet, daß die Auflageelemente (3 bis 6) in Anpassung an die zu massierenden Körperbereiche ausgebildet sind, wobei insbesondere jeweils ein Auflageelement (3, 4, 5, 6) für den Schulterbereich, den Bereich von unterem Rücken/Gesäß/Oberschenkel, den Unterschenkelbereich und ggf. den Fußbereich vorgesehen ist.

6. Massagevorrichtung nach einem der vorhergehenden Ansprüche, dadurch gekennzeichnet, daß ein Auflageelement (3, 5) für den Schulterbereich und/oder den Unterschenkelbereich eine trapezartige Form aufweist, ein Auflageelement (4) für den Bereich von unterem Rücken/Gesäß/Oberschenkel eine Form nach Art eines Doppeltrapezes aufweist und/oder ein Auflageelement (6) für den Fußbereich, insbesondere den Fußsohlenbereich, eine keilartige oder wulstartige Form aufweist.

7. Massagevorrichtung nach einem der vorhergehenden Ansprüche, dadurch gekennzeichnet, daß die mattenartige Auflage und die Auflageelemente aus unterschiedlich geformtem und/oder unterschiedlich hartem Material, insbesondere Schaumstoffmaterial, gebildet sind.

8. Massagevorrichtung nach einem der vorhergehenden Ansprüche, dadurch gekennzeichnet, daß die Mittel, die zum Aufbringen von Vibrationsbewegungen auf die Auflage vorgesehen sind, in die Auflage (2) integriert sind.

9. Massagevorrichtung nach einem der vorhergehenden Ansprüche, dadurch gekennzeichnet, daß die Auflageelemente (3 bis 6) ebenfalls Mittel zur Erzeugung von Vibrationsbewegungen aufweisen, wobei diese Mittel insbesondere in die Auflageelemente integriert sind.

10. Massagevorrichtung nach einem der vorhergehenden Ansprüche, dadurch gekennzeichnet, daß es sich bei den Mitteln zum Aufbringen bzw. zur Erzeugung von Vibrationsbewegungen um mindestens einen Elektromotor handelt, wobei dieser insbesondere auf seiner Antriebswelle mit einem Exzentergewicht versehen ist.

11. Massagevorrichtung nach einem der vorhergehenden Ansprüche, dadurch gekennzeichnet, daß in der mattenartigen Auflage oder in mindestens einem Auflageelement Mittel zur Magnetbehandlung, Mittel zur Infrarotbehandlung und/oder Heizmittel vorgesehen sind.

12. Massagevorrichtung nach einem der vorhergehenden Ansprüche, dadurch gekennzeichnet, daß die Massagevorrichtung ein Bedienelement, vorzugsweise in Form einer Handbedienung, zur Durchführung und/oder Steuerung von Massagebehandlungen aufweist, wobei insbesondere die Parameter einer solchen Behandlung anhand vordefinierter und/oder individuell einstellbarer Werte auswählbar sind.

13. Massageverfahren, bei dem die in oder an einer mattenartigen Auflage erzeugten Vibrationsbewegungen mit Hilfe von zusätzlichen separaten Auflageelementen, die an der Auflage anordenbar sind, auf mindestens einen Teil der zu massierenden Körperbereiche übertragen werden.
